(19) Europäisches Patentamt
European Patent Office
Office européen des brevets

(11) Publication number: **0 310 907 A1**

(12) **EUROPEAN PATENT APPLICATION**

(21) Application number: **88115810.9**

(22) Date of filing: **26.09.88**

(51) Int. Cl.⁴: **C07C 125/063 , C07C 127/19**

(30) Priority: **09.10.87 JP 255008/87**
**09.10.87 JP 255009/87**
**22.01.88 JP 12018/88**
**22.01.88 JP 12019/88**

(43) Date of publication of application:
**12.04.89 Bulletin 89/15**

(84) Designated Contracting States:
**DE FR GB IT NL**

(71) Applicant: **NKK CORPORATION**
**1-2, 1-chome, Marunouchi Chiyoda-ku**
**Tokyo(JP)**

(72) Inventor: **Ikariya, Takao Patent & License and Quality Standards Dept NKK CORPORATION**
**1-2 1-chome**
**Marunouchi Chiyoda-ku Tokyo(JP)**
Inventor: **Itagaki, Masanori Patent & License and Quality Standards Dept NKK CORPORATION**
**1-2 1-chome**
**Marunouchi Chiyoda-ku Tokyo(JP)**
Inventor: **Shimoyama, Izumi Patent & License and Quality Standards Dept NKK CORPORATION**
**1-2 1-chomE**
**Marunouchi Chiyoda-ku Tokyo(JP)**
Inventor: **Mizuguchi, Masatsugu Patent & License and Quality Standards Dept NKK CORPORATION**
**1-2 1-chome**
**Marunouchi Chiyoda-ku Tokyo(JP)**
Inventor: **Hachiya, Tetsuo Patent & License and Quality Standards Dept NKK CORPORATION**
**1-2 1-chome**
**Marunouchi Chiyoda-ku Tokyo(JP)**

(74) Representative: **Popp, Eugen, Dr. et al**
**MEISSNER, BOLTE & PARTNER**
**Widenmayerstrasse 48 Postfach 86 06 24**
**D-8000 München 86(DE)**

(54) **Method of manufacturing aromatic urethane.**

(57) This invention is a method of manufacturing aromatic urethane including an aromatic urea production step of reacting an aromatic primary amine, an aromatic nitro-compound, and carbon monoxide with one another, using a catalyst mainly consisting of a compound containing a platinum group metal, and using a compound having a coordination ability to the catalyst as at least part of a solvent, thereby producing N,N′-di-substituted urea, and separating and isolating the produced N,N′-di-substituted urea from the reaction solution, and further including a step of reacting the N,N′-di-substituted urea obtained in the urea production step with an organic compound containing a hydroxyl group to produce the aromatic primary amine and aromatic urethane, and separating the aromatic primary amine to produce aromatic urethane.

EP 0 310 907 A1

Xerox Copy Centre

## Method of manufacturing aromatic urethane

The present invention relates to a method of producing N,N′-di-substituted urea by reacting an aromatic primary amine, an aromatic nitro-compound, and carbon monoxide, and a method of manufacturing aromatic urethane by reacting the obtained N,N′-di-substituted urea with an organic compound containing a hydroxyl group.

U.S.P. No. 4,678,856 filed by the present inventor discloses a method of manufacturing N,N′-di-substituted urea and a method of manufacturing aromatic urethane using the N,N′-di-substituted urea. In this case, the aromatic urethane is manufactured by a 2-step reaction, i.e., N,N′-di-substituted urea is produced by reacting an aromatic primary amine, an aromatic nitro-compound, and carbon monoxide, and then aromatic urethane is manufactured by reacting the obtained N,N′-di-substituted urea with an organic compound containing a hydroxyl group. This patent specification also discloses that a compound containing a platinum group metal compound such as a ruthenium complex or a rhodium complex having no halogen ligands is used as a catalyst, and that a halogen compound need not be used as a cocatalyst. In addition, in the first N,N′-di-substituted urea production step, a reaction can be achieved, in the presence of a suitable solvent, e.g., benzene, toluene, xylene, and cyclohexane or without using a solvent, and a surplus amount of an aromatic primary amine is added as a solvent, thereby promoting the reaction. However, the solvent disclosed in this patent specification has no coordination ability to a compound mainly consisting of a platinum group metal.

U.S.P. Nos. 4,600,793, 4,603,216, 4,629,804, and 4,705,883 disclose that a nitrogen-containing an organic compound is reacted with carbon monoxide in the presence of primary amine and a catalyst substantially not containing a redox metal halide component and containing ruthenium or rhodium, thereby carbonylating the nitrogen-containing organic compound, and that no halogen compound is used as a cocatalyst. These patent specifications also disclose that the reaction is performed in the presence of a suitable solvent or without using a solvent. Examples of the solvent are an aromatic solvent such as benzene, toluene, and xylene; a nitrile such as acetonitrile and benzonitrile; a sulfone such as sulfolane; a halogenated aliphatic hydrocarbon such as 1,1,2-trichloro-1,2,2-trifluoroethane; a halogenated aromatic hydrocarbon such as monochlorobenzene, dichlorobenzene, and trichlorobenzene; a ketone; an ester; a phenol; a monohydric alcohol such as methyl, ethyl, n-propyl, butyl, amyl, hexyl, benzyl, chlorobenzyl, and methoxybenzyl alcohol; a diol such as ethylene glycol, diethylene glycol, propylene glycol, and dipropylene glycol; a triol such as glycerol, trimethylol, propane, and hexanetriol; a tetraol such as pentaerythritol; and an ether of a polyether in which at least one hydroxyl group is not etherified but left therein. However, these solvents are compounds not contributing to the reaction.

The present invention is an improvement over U.S.P. No. 4,678,856 and has as its first object to provide a method of manufacturing aromatic urethane catalyzed by a compound containing a platinum group metal compound such as a ruthenium or rhodium complex having no halogen ligand without using a halogen compound as a cocatalyst, thereby solving various problems caused by use of the halogen compound.

It is a second object of the present invention to provide a method of manufacturing aromatic urethane by a 2-step reaction, thereby improving the manufacturing yield and easily recovering a catalyst and the produced aromatic urethane.

It is a third object of the present invention to provide a method of manufacturing aromatic urethane wherein, in a urea production reaction performed in a first step, a compound having coordination ability to the catalyst is used as at least part of a solvent so that the catalyst can be stably dissolved in a solution to be efficiently recovered and reused, thereby manufacturing the aromatic urethane in a more efficient manner.

In order to achieve the above objects, the present invention comprises: a urea production step of reacting an aromatic primary amine, an aromatic nitro-compound, and carbon monoxide with each other, using a catalyst mainly consisting of a compound containing a platinum group metal and using a compound having a coordination ability to the catalyst as at least part of a solvent, thereby preparing N,N′-di-substituted urea and separating and recovering the produced N,N′-di-substituted urea from the reaction solution; and a step of reacting the N,N′-di-substituted urea obtained in the urea production step with an organic compound containing a hydroxyl group to produce the aromatic primary amine and aromatic urethane and separating the aromatic primary amine from the resultant solution to obtain aromatic urethane.

According to the present invention, the N,N-disubstituted urea produced in the first reaction has a low solubility with respect to the solvent, the aromatic primary amine, and aromatic nitro-compound as materials. For this reason, after the solution has cooled to room temperature following the first step reaction, the N,N′-di-substituted urea can then be easily crystallized as a reaction product. Therefore, by filtration of

the resultant solution, the N,N′-di-substituted urea can be efficiently separated and isolated.

The catalyst mainly consisting of the compound containing the platinum group metal used in the first reaction is present in the solution in a form stabilized by coordination of the solvent having the coordination ability to the metal. For this reason, the catalyst contained in a filtrate from which the N,N′-di-substituted urea is separated in the first step can be directly reused in the first reaction, resulting in an economical advantage over the prior arts.

In the first reaction, part or all of the solvent is a compound having the coordination ability to the catalyst. However, if necessary, part of the solvent may be a compound such as toluene or cyclohexane which does not contribute to the reaction. Alternatively, a surplus amount, which is larger than a theoretical amount contributing to the reaction, of the aromatic primary amine may be added as a reaction material to be used as part of the solvent. In this case, since a concentration of the above reaction material is increased, a reaction rate is also increased, whereby the first reaction can be performed at a high reaction rate.

Since the catalyst used in the first reaction mainly consists of the platinum group metal, a halogen compound need not be used. For this reason, problems with corrosion of the materials caused by the halogen compound can be solved, and hence expensive materials having corrosion resistance qualities with respect to a reactor used in the reaction are no longer required.

In the first reaction, the N,N′-di-substituted urea can be obtained at a high yield and with less undesirable side reaction.

A one-step reaction, so called one-pot reaction, for manufacturing aromatic urethane by use of a catalyst mainly including a palladium-halogen compound is well known in the art. However this step often encounters difficulty in the separation of catalyst from the reaction mixture and also in isolation of the reaction product. A two-step reaction (two pot reaction) according to the present invention is applicable to this manufacture, it becomes very easy to separate the palladium and halogen compound from N,N′-diphenylurea product just by filtration.

Since it is not necessary that a catalyst be used in a second reaction of the present invention, the aromatic urethane can be separated and recovered as a distilled residue after the second reaction, and the aromatic urethane need not be distilled.

While, the aromatic primary amine, which is a distilled material, and a residual hydroxyl group-containing organic compound have comparatively low boiling points, distillation can be easily performed under moderate conditions.

The aromatic primary amine recovered by distillation can be reused in the first N,N′-disubstituted urea production reaction, thereby utilizing the material to its maximum efficiency.

An undesirable side reaction is less likely to occur in the second reaction as compared with the first reaction. As a result, the aromatic urethane can be manufactured at a high yield.

The inventions recited from claim 39 onwards are featured by using a two-step method for the above-mentioned manufacture of aromatic urethane, i.e., the manufacture wherein an aromatic compound-containing nitrogen, an organic compound containing a hydroxyl group, and carbon monoxide are reacted with one another in the presence of a catalyst mainly consisting of a compound containing an element selected from a platinum group metal and selenium. In the first step of the reaction, the N,N′-di-substituted urea produced from an aromatic compound-containing nitrogen can be effectively separated from a catalyst and a cocatalyst. Thus, the use of the two-step method of the present invention eliminates the problem associated with the separation of a very small amount of catalyst dissolved in a product. Further, with the use of the two-step method of the present invention, a cocatalyst including a halogen compound or the like, which is often used in the production of aromatic urethane, can be effectively collected for reuse.

The present invention will be described in detail below.

The first reaction of an aromatic primary amine, an aromatic nitro-compound, and carbon monoxide is as follows:

$$Ar\text{-}NO_2 + ArNH_2 + 3CO \rightarrow Ar\text{-}NH\overset{\overset{\displaystyle O}{\|}}{C}NH\text{-}Ar + 2CO_2$$

Examples of the aromatic primary amine used in the reaction are anilines, aminonaphthalenes, aminoanthracenes, and aminobiphenyls. More specifically, examples of a compound are: aniline, o-, m-, and p-toluidine; o-, m-, and p-chloroaniline; α and β naphthylamine; 2-methyl-1-aminonaphthalene, diaminobenzene, triaminobenzene, aminotoluene, diaminotoluene, and aminonaphthalene; isomers of these compounds; and mixtures thereof.

Examples of the aromatic nitro-compound used in the reaction are: nitrobenzenes, nitronaphthalenes, nitroanthracenes, and nitro-compounds having at least one of the hydrogen atoms therein substituted by another substituting group such as a halogen atom, a cyano group, an alicyclic group, an alkyl group, an alkoxy group, a sulfone group, a sulfoxide group, a carbonyl group, an ester group, or an amide group. Examples of a compound are: nitrobenzene, o-, m- and p-nitrotoluene; o-nitro-p-xylene, 2-methyl-1-nytronahthalene, o-, m- and p-chloronitrobenzene; 1-bromo-4-nitrobenzene; isomers of these compounds; and mixtures thereof. Note that the nitro-compounds are preferably used in correspondence to the aromatic primary amine.

Carbon monoxide used in the reaction may be pure or contain nitrogen, argon, helium, carbon dioxide gas, or hydrocarbon.

A preferable catalyst mainly consisting of a compound containing a platinum group metal for performing the reaction is a compound of: a platinum group metal element such as ruthenium, rhodium, palladium, and platinum and a ligand such as carbon monoxide and organo phosphines including compounds represented by the formula $PR^1R^2R^3$, wherein $R^1$, $R^2$, and $R^3$ are the groups consisting of hydrogen, alkyl such as methyl, ethyl, n-propyl, buthyl, cyclohexyl, etc, and phenyl, o, m, and p-methyl-phenyl etc. Most preferably, this compound does not contain a halogen atom. Examples of the compound are ruthenium complex compounds such as $Ru_3(CO)_{12}$, $H_4Ru_4(CO)_{12}$, $[Ru_2(CO)_4(HCOO)_2]n$, $Ru(CO)_3(diphos)$, $\{Ru(CO)_2(HCOO)-PR^1R^2R^3\}_2$, and $Ru(acac)_3$; and rhodium complex compounds such as $Rh_6(CO)_{16}$, $RhH(CO)(PPh_3)_3$, $Rh-(acac)(CO)(PPh_3)$, $Rh(acac)(CO)_2$, and $Rh(acac)_3$. In the above formulas, diphos represents a bisphosphine ligand such as diphenylphosphinoethane, diphenylphosphinopropane, and diphenylphosphinobutane, and acac represents acetylacetonate. In addition to the above complex compounds, an inorganic platinum group metal compound which changes into the active species in a reactive system may be used as a catalyst of the present invention. Examples of the compound are $RuO_2 \bullet nH_2O$, Ru-black, and Ru/C. These compounds are assumed to change into a carbonyl complex exhibiting an catalytic activity for the present reaction. In addition, as the catalyst of the present invention, a combination of a platinum group metal with cobalt, iron, ruthenium, rhodium, or palladium may be used.

Part or all of the solvent used in the first reaction is a compound having the coordination ability to the catalyst. Examples of the preferable compound are: nitriles, pyridines, quinolines, carbonyl compounds and cyclic ethers, etc. More specifically, examples of the compound are: acetonitrile, benzonitrile, pyridine, aminopyridine, diaminopyridine, quinoline, N,N,N′,N′-tetramethyl urea, tetrahydrofuran, 1-4-dioxane, isomers of these compounds, and mixtures thereof. In order to stably dissolve the catalyst in the solvent, 1 mℓ to 20 mℓ of a solvent having coordination ability is preferably contained with respect to $5 \times 10^{-4}$ g atom-Ru of a catalyst.

When the compound having the coordination ability to the catalyst is used as part of the solvent, the rest of the solvent may be a compound such as toluene or cyclohexane which does not contribute to the reaction. Alternatively, a surplus amount, i.e., an amount larger than a theoretical amount which is required for the reaction, of an aromatic primary amine may be added so that the aromatic primary amine is substantially used as the rest of solvent. The reaction materials and the compound not contributing to the reaction may be simultaneously used as the rest of the solvent. Normally, a total amount of the solvent is preferably 30 mℓ to 1 ℓ with respect to a total amount 6.5 g of the reaction materials of nitrobenzene and aniline (1/1 molar ratio).

A reaction temperature in the first step is generally 30°C to 300°C, and preferably, 120°C to 200°C. A reaction pressure is 1 $kg/cm^2$ to 500 $kg/cm^2$, and preferably, 1 $kg/cm^2$ to 150 $kg/cm^2$. A reaction time is normally several minutes to several hours, depending on other reaction conditions.

The aromatic urea obtained in the first reaction has a low solubility with respect to the solvent, the aromatic amine, and the aromatic nitro-compound. For this reason, by only cooling the solution to about a room temperature after the reaction, the produced aromatic urea compound separates from the solution into individual crystals. Therefore, by filtration of the resultant solution, the aromatic urea can be efficiently obtained as a solid product. Meanwhile, the catalyst having catalyltic reactivity is stabilized by the solvent having the coordination ability present in the reaction system and therefore does not separate from the filtrate, but remains present therein. Therefore, this catalyst can be directly reused in the first reaction.

After the reaction mixture has been solidified and separated by cooling at a room temperature, it is then washed by a solvent such as toluene or benzene. As a result, since all the components have been separated and removed except for N,N′-di-substituted urea, only the N,N′-di-substituted urea can be isolated.

Then, the N,N′-di-substituted urea obtained in the first reaction is reacted with an organic compound containing a hydroxyl group as follows, thereby forming an aromatic primary amine and aromatic urethane:

$$Ar\text{-}NH\overset{O}{\overset{\|}{C}}NH\text{-}Ar + ROH \rightarrow Ar\text{-}NHCOOR + Ar\text{-}NH_2$$

Examples of the organic compound containing a hydroxyl group are monohydric alcohols and monohydric phenols. More specifically, examples of the compound are: monohydric alcohols such as methyl, ethyl, n-propyl, isopropyl, n-butyl, isobutyl, and t-butyl, and alkylphenols such as phenol, chlorophenol, methyl, n-propyl, and isopropyl.

A reaction temperature in the second step is normally 80°C to 300°C, and preferably, 120°C to 200°C. A pressure is generally an autogenous pressure with respect to a reaction temperature of the organic compound containing a hydroxyl group and the solvent to be used.

This reaction can be performed using no catalyst.

After the reaction, distillation is performed such that the aromatic urethane is recovered as a distilled residue and the aromatic primary amine is recovered as a distillate. This aromatic primary amine can be reused in the first N,N′-di-substituted urea production reaction.

The first and second reactions are combined as follows, and aromatic urethane is obtained by a reductive carbonylation reaction of nitrobenzene:

$$Ar\text{-}NO_2 + ArNH_2 + 3CO \rightarrow Ar\text{-}NH\overset{O}{\overset{\|}{C}}NH\text{-}Ar + 2CO_2$$

$$Ar\text{-}NH\overset{O}{\overset{\|}{C}}NH\text{-}Ar + ROH \rightarrow Ar\text{-}NHCOOR + Ar\text{-}NH_2$$

$$\overline{Ar\text{-}NO_2 + 3CO + ROH \rightarrow Ar\text{-}NHCOOR + 2CO_2}$$

## Examples

Examples of the present invention will be described in detail below. In the following examples, in place of the conventional term "urethane", the term "carbamic acid ester" is used to more clearly represent the make-up of the compound.

### Example 1

3.69 g of nitrobenzene, 40 mℓ of aniline, 1.0 mℓ of pyridine, and 0.10 g of $Ru_3(CO)_{12}$ were supplied into an autoclave type of reaction vessel having a volume of 200 mℓ. The interior of a system was replaced with carbon monoxide at 50 kg/cm$^2$ at room temperature. Then, the materials were heated to 160°C with stirring for 2.0 hours. After the reaction, the autoclave was cooled to room temperature, the interior of the autoclave was exhausted, and the reaction mixture was then filtered from the filtrate, thereby obtaining 5.33 g of N,N′-diphenyl urea. The filtrate was analyzed by liquid chromatography (HPLC) and gas chromatography (GC), 0.02 g of N,N′-diphenyl urea were found to be contained therein, and no nitrobenzene was detected.

The yield of the isolated N,N′-diphenyl urea was 84%, and the combined yields of the isolated and non-isolated (i.e., present in the solution) N,N′-diphenyl urea was 87%.

Then, 3.00 g of crystals of the isolated N,N′-diphenyl urea and 50.0 g of methyl alcohol were supplied into another reaction vessel having a volume of 200 mg and were caused to react with each other at 160°C for 3 hours. After the reaction, the reaction solution was analyzed. As a result, the yield of methyl N-phenyl carbamate was 94%, and that of aniline was 95%.

An N,N′-diphenyl urea production test was conducted following the same procedures as in Example 1 except that the amounts of materials used and the type and amount of the solvent used were changed. The reaction conditions and reaction results are shown in Table 1.

### Example 10

An N,N′-diphenyl urea production test was conducted following the same procedures as in Example 1 except that the type of catalyst was changed to $[Ru_2(CO)4(HCOO)_2]n$. Reaction conditions and reaction results are shown in Table 2.

Example 11

An N,N-diphenyl urea production test was conducted following the same procedures as in Example 1 except that the type of catalyst was changed to $[RuO_2 \bullet nH_2O]$ (available from Nippon Engel Hald Co.; Ru content = 47%). The reaction conditions and reaction results are shown in Table 2.

When the N,N′-diphenyl urea obtained in the reactions in Examples 2 to 11 were reacted with methyl alcohol following the same procedures as in Example 1, the yield of methyl N-phenyl carbamate was 92 to 96%.

Example 12

40 mℓ of aniline, 0.05 g of Pd black, 0.40 g of potassium iodide were supplied into an autoclave type of reaction vessel having a volume of 200 mℓ, and the interior of a system was replaced with oxygen. Oxygen was supplied into the autoclave to obtain 2.5 kg/cm², and then carbon monoxide to obtain a total pressure of 50 kg/cm². Then, the reaction was carried out with stirring at 160° C for 2.0 hours. After the reaction, the autoclave was cooled to room temperature, the interior of the autoclave was exhausted. Thereafter, the reacted solution was filtered, thereby obtaining a mixture containing crystals of N,N′-diphenyl urea and a catalyst. The mixture was dissolved in organic solvent such as acetone and then filtered, to remove the insoluble catalyst. The filtered mixture was condensed, thereby obtaining 6.5 g of crystalline N,N′-diphenyl urea. An elemental analysis showed that the obtained N,N′-diphenyl urea did not contain a halogen element or palassium. When the N,N′-diphenyl urea was reacted with methyl alcohol following the same procedures as in Example 1, the yield of methyl N-phenyl carbamate was 94%.

Example 13

An N,N′-diphenyl urea production test was conducted, following the same procedures as in Example 12 except that the type of catalyst was changed to 5%Pd-carbon. In this case, the yield of N,N′-diphenyl urea was 6.90 g. An elemental analysis showed that the obtained N,N′-diphenyl urea did not contain a halogen element or palassium. When the N,N′-diphenyl urea was reacted with methyl alcohol following the same procedures as in Example 1, the yield of methyl N-phenyl carbamate was 95%.

Comparative Example 1

6.12 g of nitrobenzene, 37.0 g of methanol, and 0.11 g of $Ru_3(CO)_{12}$ were supplied into an autoclave type of reaction vessel having a volume of 200 mℓ, the interior of a system was replaced with carbon monoxide, and carbon monoxide was supplied into the autoclave to obtain 50 kg/cm². Then, while the materials were being stirred by the magnetic stirring, they were caused to react with each other at 160° C for 5.0 hours. After the reaction, the resultant solution was analyzed by liquid chromatography (HPLC). As a result, the conversion of nitrobenzene was 32%, the selectivity coefficient of methyl N-phenyl carbamate was 13%, and the selectivity coefficient of by-product aniline was 40%. That is, the yield of methyl N-phenyl carbamate was 4%, and the yield of by-product aniline was 13%.

Comparative Example 2

4.63 g of aniline, 6.12 g of nitrobenzene, 37.0 g of methanol, and 0.11 g of $Ru_3(CO)_{12}$ were supplied into an autoclave type of reaction vessel having a volume of 200 mℓ, the interior of a system was substituted by carbon monoxide, and carbon monoxide was injected into the autoclave to obtain 50 kg/cm². Then, the reaction was carried out at 160° C for 5.0 hours. After the reaction, the resultant solution was analyzed by liquid chromatography (HPLC). As a result, the yield of methyl N-phenyl carbamate based on

nitrobenzene was 61%, and the yield of N,N′-diphenyl urea was 4%.

When the resultant solution was left to stand in a freezer at -5°C for over 24 hours, no crystal separation occurred.

Table 1

| Example | Supply Amount | | | | | $PhNO_2$ | $(PhNH)_2CO$ | $(PhNH)_2CO$ |
|---|---|---|---|---|---|---|---|---|
| | $Ru_3(CO)_{12}$ | $PhNO_2$ | $PhNH_2$ | Coordination Solvent | | Conversion | Yield | Selectivity Coefficient |
| | (g) | (g) | (mℓ) | (mℓ) | | (%) | (%) | (%) |
| 1 | 0.10 | 3.69 | 40 | Pyridine | 1 | 100 | 87 | 87 |
| 2 | 0.10 | 3.63 | 30 | | 10 | 93 | 75 | 81 |
| 3 | 0.10 | 3.72 | 20 | | 20 | 72 | 55 | 76 |
| 4 | 0.10 | 3.69 | 40 | Acetonitrile | 1 | 100 | 90 | 90 |
| 5 | 0.10 | 3.70 | 30 | | 10 | 100 | 91 | 91 |
| 6 | 0.10 | 3.71 | 20 | | 20 | 100 | 90 | 90 |
| 7 | 0.10 | 3.70 | 2.7 | | 40 | 55 | 41 | 75 |
| 8 | 0.10 | 3.68 | 40 | Benzonitrile | 1 | 100 | 92 | 92 |
| 9 | 0.10 | 3.61 | 30 | | 10 | 100 | 89 | 89 |
| 10 | 0.10 | 3.69 | 20 | | 20 | 96 | 84 | 87 |
| Reaction Conditions; CO 50 kg/cm², 160°C, 2.0 Hours * CO 50 kg/cm², 160°C, 6 Hours | | | | | | | | |

Table 2

| Example | Supply Amount | | | | | | $PhNO_2$ | $(PhNH)_2CO$ | $(PhNH)_2CO$ |
|---|---|---|---|---|---|---|---|---|---|
| | Catalyst | | $PhNO_2$ | $PhNH_2$ | Coordination Solvent | | Conversion | Yield | Selectivity Coefficient |
| | (g) | | (g) | (mℓ) | (mℓ) | | (%) | (%) | (%) |
| 11 | A | 0.10 | 3.69 | 40 | Pyridine | 1 | 100 | 92 | 92 |
| 12 | B | 0.10 | 3.68 | 40 | Pyridine | 1 | 100 | 91 | 91 |
| A ; $[Ru_2(CO)_4(HCOO)]_n$, B ; $RuO_2 \bullet nH_2O$<br>Reaction Conditions; CO 50 kg/cm², 160°C, 2.0 Hours | | | | | | | | | |

## Claims

1. A method of manufacturing aromatic urethane, comprising the steps of:

a. a urea production step of reacting an aromatic primary amine, an aromatic nitro-compound, and carbon monoxide with one another, using a catalyst mainly consisting of a compound containing a platinum group metal and using a compound having a coordination ability to said catalyst as at least part of a solvent, thereby producing N,N′-di-substituted urea, and separating and recovering the produced N,N′-di-substituted urea from the reaction solution; and

b. a step of reacting said N,N′-di-substituted urea obtained in said urea production step with an organic compound containing a hydroxyl group to produce said aromatic primary amine and aromatic urethane, and separating said aromatic primary amine to obtain aromatic urethane.

2. A method according to claim 1, characterized by further comprising the step of reusing said catalyst from which said N,N′-di-substituted urea has been separated and a filtrate containing a solvent having a coordination ability to said catalyst in said N,N′-di-substituted urea production step.

3. A method according to claim 1, characterized in that said aromatic primary amine is a compound selected from the group consisting of: anilines, aminonaphthalenes, aminoanthracenes, aminobiphenyls, and mixtures thereof.

4. A method according to claim 1, characterized in that said aromatic nitro-compound is a compound selected from the group consisting of: nitrobenzenes, nitronaphthalenes, nitroanthracenes, nitrobiphenyls, and a nitro-compound having at least one of the hydrogen atoms therein substituted by a halogen atom, a cyano group, an alicyclic group, an aromatic group, an alkyl group, an alkoxy group, a sulfone group, a sulfoxide group, a carbonyl group, an ester group, or an amide group.

5. A method according to claim 1, characterized in that said solvent of a compound having the coordination ability to said catalyst is a compound selected from the group consisting of: nitrils, pyridines, quinolines, cyclic ethers, and mixtures thereof.

6. A method according to claim 1, characterized in that said compound containing a platinum group metal is a compound of a material selected from the group consisting of organic metal compounds having a ligand and an organic group with a platinum group metal, and which does not contain a halogen element.

7. A method according to claim 6, characterized in that said compound containing a platinum group metal is a compound selected from the group consisting of: a ruthenium complex compound, a rhodium complex compound, and mixtures thereof.

8. A method according to claim 1, characterized in that said compound having the coordination ability to said catalyst is a compound selected from the group consisting of: pyridine, acetonitrile, benzonitrile, and mixtures thereof.

9. A method according to claim 1, characterized in that said solvent essentially consists of said compound having the coordination ability to said catalyst.

10. A method according to claim 1, characterized in that said solvent essentially consists of said compound having the coordination ability to said catalyst, and the surplus amounts of an aromatic primary amine which are larger than the theoretical amounts thereof contributing to the reaction of the step a of the claim 1.

11. A method according to claim 1, characterized in that said solvent essentially consists of said compound having the coordination ability to said catalyst, and a compound not contributing to the reaction of the step a of the claim 1.

12. A method according to claim 1, characterized in that said solvent essentially consists of: said compound having the coordination ability to said catalyst; the surplus amounts of an aromatic primary amine which are larger than theoretical amounts thereof contributing to the reaction of the step a of the claim 1; and a compound not contributing to the reaction.

13. A method according to claim 1, characterized in that 1 mℓ to 20 mℓ of said compound having the coordination power with respect to said catalyst are contained with respect to $5 \times 10^{-4}$ g atom-Ru of said catalyst.

14. A method according to claim 1, characterized in that 30 mℓ to 1 ℓ of said solvent are contained with respect to a total amount 6.5 g of the reaction materials of nitrobenzene and aniline (1/1 molar ratio).

15. A method according to claim 1, characterized in that said urea production step is performed at a reaction temperature of 30°C to 300°C and a reaction pressure of 1 kg/cm$^2$ to 500 kg/cm$^2$.

16. A method according to claim 1, characterized in that said urea production step is performed at a temperature of 120°C to 200°C and a reaction pressure of 1 kg/cm$^2$ to 150 kg/cm$^2$.

17. A method according to claim 1, characterized by further comprising the step of reusing said aromatic primary amine separated in said step of obtaining aromatic urethane in said N,N′-di-substituted urea production step.

18. A method according to claim 2, characterized by further comprising the step of reusing said aromatic primary amine separated in said step of obtaining aromatic urethane in said N,N′-di-substituted urea production step.

19. A method according to claim 1, characterized in that no catalyst is used in the reaction of obtaining aromatic urethane.

20. A method according to claim 1, characterized in that said organic compound containing a hydroxyl group is a compound selected from the group consisting of: monohydric alcohols, monohydric phenols, and mixtures thereof.

21. A method according to claim 1, characterized in that said method is performed at a reaction temperature of 80 to 300°C and at an autogenous pressure with respect to this temperature.

22. A method according to claim 1, characterized in that said method is performed at a temperature of 120 to 200° C and at an autogenuous pressure with respect to this temperature.

23. A method of manufacturing a urea, comprising the steps of:

a. reacting an aromatic primary amine, an aromatic nitro-compound, and carbon monoxide with one another, using a catalyst mainly consisting of a compound containing a platinum group metal and using a compound having a coordination ability to said catalyst as at least a part of the solvent, thereby producing a urea; and

b. separating and recovering the produced urea from the reaction solution.

24. A method according to claim 23, characterized by further comprising the step of reusing said catalyst from which said urea has been separated and a filtrate containing a solvent having a coordination ability to said catalyst in said urea production step.

25. A method according to claim 23, characterized in that said aromatic primary amine is a compound selected from the group consisting of: anilines, aminonaphthalenes, aminoanthracenes, aminobiphenyls, and mixtures thereof.

26. A method according to claim 23, characterized in that said aromatic nitro-compound is a compound selected from the group consisting of: nitrobenzenes, nitronaphthalenes, nitroanthracenes, nitrobiphenyls, and a nitro-compound having at least one of the hydrogen atoms therein substituted by a halogen atom, a cyano group, an alicyclic group, an aromatic group, an alkyl group, an alkoxy group, a sulfone group, a sulfoxide group, a carbonyl group, an ester group, or an amide group.

27. A method according to claim 23, characterized in that said solvent of a compound having the coordination power with respect to said catalyst is a compound selected from the group consisting of: nitrils, pyridines, quinolines, and cyclic ethers.

28. A method according to claim 23, characterized in that said compound containing a platinum group metal is a compound of a material selected from the group consisting of organic metal compounds having a ligand and an organic group with a platinum group metal, and which does not containing halogen element.

29. A method according to claim 28, characterized in that said compound containing a platinum group metal is a compound selected from the group consisting of: a ruthenium complex compound, a rhodium complex compound, and mixture thereof.

30. A method according to claim 23, characterized in that said compound having the coordination ability to said catalyst is a compound selected from the group consisting of: pyridine, acetonitrile, and benzonitrile, and mixtures thereof.

31. A method according to claim 23, characterized in that said solvent essentially consists of said compound having the coordination ability to said catalyst.

32. A method according to claim 23, characterized in that said solvent essentially consists of said compound having the coordination ability to said catalyst, and a surplus amount of an aromatic primary amine.

33. A method according to claim 23, characterized in that said solvent essentially consists of said compound having the coordination ability to said catalyst, and a compound not contributing to the reaction.

34. A method according to claim 23, characterized in that said solvent essentially consists of said compound having the coordination ability to said catalyst, an aromatic primary amine, and a compound not contributing to the reaction.

35. A method according to claim 23, characterized in that 1 mℓ to 20ℓ of said compound having the coordination power with respect to said catalyst are contained in the solvent with respect to 5 x $10^{-4}$ g atom-Ru of said catalyst.

36. A method according to claim 23, characterized in that 30 mℓ with 1 mℓ of said solvent are contained in the solvent with respect to a total amount 6.5 g of the reaction materials of nitrobenzene and aniline (1/1 molar ratio).

37. A method according to claim 23, characterized in that said urea production step is performed at a reaction temperature of 30° C to 300° C and a reaction pressure of 1 kg/cm² to 500 kg/cm².

38. A method according to claim 23, characterized in that said urea production step is performed at a temperature of 120° C to 200° C and a reaction pressure of 1 kg/cm² to 150 kg/cm².

39. A method of manufacturing aromatic urethane, comprising the steps of:

a. a urea production step of reacting an aromatic compound-containing nitrogen, and carbon monoxide with one another, using a catalyst mainly consisting of a compound containing an element selected from the group consisting of a platinum group metal and selenium, thereby producing N,N′-di-substituted urea, and separating and recovering the produced N,N′-di-substituted urea from the reaction solution; and

b. a step of reacting said N,N′-di-substituted urea obtained in said urea production step with an organic compound containing a hydroxyl group to produce said aromatic primary amine and aromatic urethane, and separating said aromatic primary amine to obtain aromatic urethane.

40. A method according to claim 1, characterized by further comprising the step of reusing said catalyst from which said N,N′-di-substituted urea has been separated in said N,N′-di-substituted urea production step.

41. A method according to claim 1, characterized in that said aromatic nitrogen-containing compound is selected from the group consisting of: anilines, aminonaphthalenes, aminoanthracenes, aminobiphenyls, mixtures thereof, nitrobenzenes, nitronaphthalenes, nitroanthracenes, nitrobiphenyls, and a nitro-compound having at least one of the hydrogen atoms therein substituted by a halogen atom, a cyano group, an alicyclic group, an aromatic group, an alkyl group, an alkoxy group, a sulfone group, a sulfoxide group, a carbonyl group, an ester group, or an amide group.

42. A method according to claim 39, characterized in that no catalyst is used in the reaction N,N′-di-substituted urea and the organic compound containing a hydroxyl group.

European Patent Office

# EUROPEAN SEARCH REPORT

Application number: EP 88115810.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | US - A - 4 603 216 (JOHN H. GRATE et al.) * Abstract * -- | 1 | C 07 C 125/063<br>C 07 C 127/19 |
| D,A | US - A - 4 600 793 (JOHN H. GRATE et al.) * Abstract * -- | 1 | |
| D,A | US - A - 4 678 856 (TAKAO IKARIYA et al.) * Abstract; pages 3,4 * -- | 1,23, 39 | |
| D,A | EP - A1 - 0 195 515 (E. I. DU PONT DE NEMOURS AND COMPANY) * Abstract * -- | 1 | **TECHNICAL FIELDS SEARCHED (Int. Cl.4)** |
| P,A | EP - A2 - 0 250 037 (SHELL INTERNATIONALE RESEARCH MAATSCHAPPIJ B.V.) * Claims 1,7 * -- | 1,23, 29 | C 07 C 125/00<br>C 07 C 127/00 |
| A | DE - A1 - 3 405 582 (MTA KÖZPONTI KEMIAI KUTATO INTEZETE) * Abstract * ---- | 1,23, 39 | |

The present search report has been drawn up for all claims

| Place of search | Date of completion of the search | Examiner |
|---|---|---|
| VIENNA | 04-01-1989 | HEIN |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document